# EUROPEAN PATENT SPECIFICATION

(11) **EP 1 980 640 B1**
(45) Date of publication and mention of the grant of the patent: **21.01.2015**
(21) Application number: 08007050.1
(22) Date of filing: 09.04.2008
(51) Int. Cl.: C23C 8/02, C23C 8/08, C23C 8/20, A61L 27/06, C23C 8/36, C21D 1/06, C21D 1/78, C22F 1/18

(54) **Surface carburization technique of medical titanium alloy femoral head in total hip arthroplasty**
Oberflächenaufkohlungsverfahren eines medizinischen Femurkopfs aus einer Titanlegierung in einer vollständigen Hüftarthroplastik
Technique de carburation de surface de tête fémorale d'alliage de titane médical dans une arthroplastie totale de la hanche

(30) Priority: 10.04.2007 CN 200710020889
(43) Date of publication of application: 15.10.2008
(73) Proprietor: China University of Mining and Technology, Quanshan district Xuzhou Jiangsu 221116 (CN)
(72) Inventor: Ge, Shirong China University of Mining and Technology, Jiangsu 221116 (CN); Luo, Yong China University of Mining and Technology, Jiangsu 221116 (CN)
(74) Representative: Fuchs Patentanwälte Partnerschaft mbB

(56) References cited:
- EP-A1- 0 485 152
- WO-A1-02/053792
- JP-A- 63 157 852
- JP-A- 2004 010 979
- US-A- 4 687 487

## Description

The present invention relates to a surface carburization technique of medical titanium alloy, especially of a medical titanium alloy femoral head in total hip arthroplasty, particularly preparation of low-wearing titanium alloy femoral head in total hip arthroplasty used in replacement of human body total hip or knee arthroplasty.

Since Branemark adopted titanium alloy as oral implant in sixties of 20th century, titanium alloy has ended history as a material solely for aerospace application, and gained wide development and application in the field of biomedical material. Presently, Ti6Al4V alloy, also called TC4 titanium alloy, is the most widely used material in orthopedics. It has excellent corrosion resistance, very good biocompatibility, high mechanical strength, and good machinability; therefore it is widely used as restoration material for surgery. But, research has shown Ti6Al4V alloy has low plastic shear resistance and undesirable work-hardening property, which is insufficient to resist friction wearing due to mechanical property impact; and the TiO2 film on surface is prone to peeling, and can not well protect the subsurface layer. Usually under normal condition, the titanium alloy will form a stable and continuous oxide passivation film on surface which possesses good corrosion resistance, but due to complexity of human body environment and erosion of external force and body fluid, the surface passivation film could be peeled and dissolved; therefore some substances will be released into the tissue in use. Moreover, the titanium alloy has large friction coefficient, which leads to poor wearing resistance; therefore large amount of Ti, Al and V black scraps will be generated due to wearing of the implant, and those scraps can cause sterile loosening and lead to failure of the joint replacement.

For improving performance of the medical titanium alloy, various surface treatment method can be used to modify titanium alloy surface to make it more suitable for medical application. The surface modification of the titanium alloy not only keeps characteristics of the titanium alloy as a basal body material, but also dramatically improves overall performance of the titanium alloy; thus becoming hot spot in research of medical titanium alloy. As the development of ion implantation, plasma spraying, chemical plating, ion plating, PVD, CVD, microarc oxidation, and laser fusion coating techniques, TiN, TiC, DLC, and TiO2 ceramic coat with good resistance to wearing and corrosion can be formed on the titanium alloy surface to improve surface wearing resistance and corrosion resistance; HA or BG bioactive coat also can be formed on surface of titanium alloy to prevent V and Al ions in the titanium alloy from releasing into physiological environment, so as to improve biocompatibility of the material. Thus it is important to research surface modification technique of titanium alloy, prepare cermet with resistances to wearing and corrosion, and research its biofriction properties in physiological environment for developing high performance artificial joint, prolonging service life of titanium alloy artificial joint, disclosing its lubrication mechanism, and further improving stability and reliability of the artificial joint replacement.

From abstract of JP 2004 010979 A a method and apparatus for plasma carburization treatment is known. The method is carried out while adjusting a temperature to obtain an age-hardenable surface.

The abstract of JP 63 157852 A relates to a method for carburizing Ti6Al4V alloy at temperatures of about 1050°C performed in a high degree vacuum. The coated material is useful such as engine valves.

EP 0 485 152 A1 describes a method of surface hardening titanium and titanium alloy substrates. Thereby, the process temperature is in the range of about 400°C to 700°C and the process pressure is within the range of 108 to 115 kPa. Substrates to be used comprise orthopedic implant devices.

US 4 687 487 A relates to a joint implant having a hard material coating thereon. The hardening material can be selected from the group consisting of titanium carbide, titanium nitride and titanium carbon nitride.

WO 02/053792 Al relates to a process for the surface treatment of an item with a titanium surface comprising a heating step under reduced pressure. The carburizing gases are described as methane and acetylene.

The object of the present invention is to provide an alternative surface carburization technique for medical titanium alloy for obtaining deeper case hardened layers and for improving the wear resistance of respective medical implants and in particular the biofriction properties in physiological environments.

The above object and aim of the invention can be achieved with the features defined in claim 1. Preferred embodiments of the invention are defined in sub-claims.

Hence, the invention relates to a method for carburizing surfaces of medical implants of medical titanium alloy Ti₆Al₄V, comprising the steps
a) cleaning the surface,
b) placing the alloy in a vacuum carburization furnace, evacuating said carburization furnace to a pressure of 1 Pa-200 Pa,
c) heating said carburization furnace to a temperature in the range of 1100 to 1450°C and holding said temperature constant,
d) introducing C₂H₂ into the vacuum carburization furnace until the pressure in the furnace is of 1000 - 3500 Pa,
e) evacuating and discharging the gas resulting from C₂H₂ decomposition,
f) stopping heating and continuously evacuating the furnace, and
g) taking the titanium alloy out of the carburization furnace.

Preferably, the steps d) and e) are repeated at least once. In a further preferred embodiment these steps are repeated at least twice, especially at least three times, wherein a repetition of four times or even more is mostly preferred. These steps are repeated in a time interval of at least 10 minutes, preferably at least 15 minutes, whereby a repetition time of at least 20 minutes is especially preferred. According to the invention it has been shown that a repetition time of 30 minutes, especially 30 minutes ± 5 minutes leads to satisfactory results.

After final step e) the heating is stopped and the furnace is continuously further evacuated until the temperature reaches 80°C or below. Preferably, the evacuation is continued until 50°C, especially until 30°C is obtained. Usually evacuation and cooling is continued until about room temperature is achieved. Thereafter, the evacuation is stopped and the treated titanium alloy is taken out the furnace. The surface is then usually polished to achieve a smooth surface. The surface thus obtained is a TiC-ceramic.

The alloys carburized according to the method of the invention are a TC4-alloy for medical purposes. The method is usually employed to medical devices like implants, especially artificial joints or parts thereof like femoral heads in hip arthroplasty or for knee arthroplasty.

When the alloy is placed in the carburization furnace in step b) the pressure preferably is evacuated until it reaches a value of at least 200, preferably at least 150 Pa, whereby a pressure of at least 120, especially at least 100 Pa is mostly preferred. The lower limits of evacuation pressures are about 1 Pa, especially 5 Pa and preferably 10 Pa.

The temperature of the carburization furnace is in the range between 1000 and 1450 °C. Preferred lower limits of the furnace temperature are 1100°C, especially 1150°C, whereby a lower limit of 1200°C is especially preferred. Preferred upper limits are 1420°C, especially 1400°C, whereby 1370°C is a mostly preferred upper limit.

C₂H₂ must be introduced in such an amount to give a reaction with the alloy surface high enough to obtain a sufficient yield of TiC-ceramic. Preferred lower pressure limits of C₂H₂ are about 1000 Pa, especially about 1400 Pa, whereby a lower limit by minimum pressure of 1600 Pa or 1800 Pa is mostly preferred. The upper limits of the pressure until which C₂H₂ is introduced in a vacuum furnace is preferably at least 3500, especially 3000 Pa, whereby an upper limit of 2800, preferably 2200 Pa is mostly preferred. It has been found that upper limits of 2300 and 2200 Pa lead to sufficient results. A typical pressure obtained after introducing C₂H₂ is about 2000 Pa, especially 2000 ± 100 Pa.

The cleaning of the surface can be conducted by any means like a detergent-water-mixture or an organic solvent. Preferably, cleaning is performed with acetone or a solution containing the same. Preferably, the cleaning is conducted as ultrasonic treatment. The treatment time depends on the desired cleaning result. A typical treatment time is at least 10, preferably at least 15 minutes, whereby at least 20 minutes or even 45 minutes are mostly preferred. Typical cleaning times have shown to be about 30 ± 10 minutes, preferably ± 5 minutes.

The thickness of the TiC layer obtained according to the invention is usually at least 10 micron, preferably at least 50 micron. Normally, the layer has a thickness of at least 80, preferably at least about 100 micron.

A typical process leading to satisfying results comprises
a) firstly placing medical titanium alloy TC4 femoral head in total hip arthroplasty in acetone solution, and ultrasonic cleaning for 30 min,
b) placing the medical titanium alloy TC4 femoral head in total hip arthroplasty in vacuum carburization furnace, evacuating to about 100 Pa,
c) heating to about 1300 °C, and holding the temperature constant,
d) introducing C₂H₂ to reach pressure of about 2000 Pa in the furnace, and stopping introduction of C₂H₂,
e) evacuating, and discharging gas resulted from C₂H₂ decomposition,
f) repeating d) and e) every about 30 min for 4 times, stopping heating, and continuously evacuating,
g) stopping evacuation until furnace temperature cooled to about 25 °C, and taking off the medical titanium alloy TC4 femoral head in total hip arthroplasty,
h) surface polishing to give medical titanium alloy TC4 femoral head in total hip arthroplasty with TiC ceramic formed on surface thereof.

The inventive surface carburization technique of medical titanium alloy femoral head in total hip arthroplasty performs surface carburization to medical titanium alloy TC4 by using acetylene as carburizing agent to carry out gaseous carburization at high temperature to give medical titanium alloy femoral head in total hip arthroplasty with TiC ceramic on surface thereof. The TiC ceramic layer on femoral head can be more than 100 micron thick, which is relatively thick, overcomes the disadvantages in available medical titanium alloy material, and is particularly useful for replacement of total hip or knee arthroplasty. It has the advantages of low wearing, good biocompatibility, good corrosion resistance, simple preparation, low cost, and wide practicability in the technical field.

The embodiment comprises selecting medical titanium alloy TC4, mechanically processing the titanium alloy into femoral head in total hip arthroplasty with various diameters suitable for human body total hip arthroplasty, ultrasonic cleaning in acetone for 30 min, placing in high temperature carburization furnace, evacuating to 100 Pa, heating to 1300° C, and holding the temperature constant; immediately introducing C₂H₂ into the furnace cavity to reach pressure of 2000 Pa, stopping introduction of C₂H₂, evacuating, discharging the gas resulted from C₂H₂ decomposition; repeating the above process every 30 min, holding temperature for 2hr (i.e. after introducing C₂H₂ for four times), stopping heating, stopping evacuation after cooled to room temperature, and taking off the titanium alloy femoral head in total hip arthroplasty; polishing the surface of the femoral head in total hip arthroplasty, and sterilizing for use in clinical application.

## Claims

1. A method for carburizing surfaces of medical implants of medical titanium alloy Ti₆Al₄V, comprising the steps
a) cleaning the surface,
b) placing the alloy in a vacuum carburization furnace, evacuating said carburization furnace, to a pressure of 1 Pa-200 Pa,
c) heating said carburization furnace up to a temperature of 1000 - 1450 °C and holding said temperature constant,
d) introducing C₂H₂ into the vacuum carburization furnace until the pressure in the furnace is of 1000 - 3500 Pa,
e) evacuating and discharging the gas resulting from C₂H₂ decomposition,
f) stopping heating and continuously evacuating the furnace, and
g) taking the titanium alloy out of the carburization furnace.

2. Method according to claim 1 **characterised in that** steps d) and e) are repeated.

3. Method according to anyone of the preceding claims **characterised in that** the evacuation according to step e) is continued until the temperature is cooled to at least 80°C.

4. Method according to anyone of the preceding claims **characterised by** h) polishing the surface.

5. Method according to anyone of the preceding claims **characterised in that** the medical titanium alloy is a femoral head for hip arthroplasty or an artificial joint for knee arthroplasty.

6. Method according to anyone of the preceding claims **characterised in that** the surface obtained is a TiC ceramic.

7. Method according to anyone of the preceding claims **characterised in that** the surface is cleaned with an acetone solution.

8. Method according to anyone of the preceding claims **characterised in that** the cleaning is obtained by ultrasonic treatment.

9. Method according to claim 8 **characterised in that** the ultrasonic treatment is conducted for at least 15 minutes.

## Patentansprüche

1. Verfahren zum Karbonisieren von Oberflächen medizinischer Implantate aus medizinischer Titanlegierung Ti₆Al₄V, umfassend die Schritte
a) Reinigen der Oberfläche,
b) Einbringen der Legierung in einen Vakuumkarbonisierungsofen, Evakuieren des Karbonisierungsofens auf einen Druck von 1 Pa - 200 Pa,
c) Erhitzen des Karbonisierungsofens bis zu einer Temperatur von 1000 - 1450 °C und Konstanthalten dieser Temperatur,
d) Einleiten von C₂H₂ in den Vakuumkarbonisierungsofen bis der Druck in dem Ofen 1000 - 3500 Pa beträgt,
e) Evakuieren und Verwerfen des aus der C₂H₂-Zersetzung erhaltenen Gases,
f) Stoppen der Erhitzung und kontinuierliches Evakuieren des Ofens, und
g) Herausnehmen der Titanlegierung aus dem Karbonisierungsofen.

2. Verfahren nach Anspruch 1, **dadurch gekennzeichnet, dass** die Schritte d) und e) wiederholt werden.

3. Verfahren nach einem der vorhergehenden Ansprüche, **dadurch gekennzeichnet, dass** die Evakuierung gemäß Schritt e) fortgesetzt wird, bis die Temperatur auf mindestens 80°C abgekühlt ist.

4. Verfahren nach einem der vorhergehenden Ansprüche, **gekennzeichnet durch** h) Polieren der Oberfläche.

5. Verfahren nach einem der vorhergehenden Ansprüche, **dadurch gekennzeichnet, dass** die medizinische Titanlegierung ein Femurkopf zur Hüftarthroplastie oder ein künstliches Gelenk zur Kniearthroplastie ist.

6. Verfahren nach einem der vorhergehenden Ansprüche, **dadurch gekennzeichnet, dass** die erhaltene Oberfläche eine TiC-Keramik ist.

7. Verfahren nach einem der vorhergehenden Ansprüche, **dadurch gekennzeichnet, dass** die Oberfläche mit einer Acetonlösung gereinigt wird.

8. Verfahren nach einem der vorhergehenden Ansprüche, **dadurch gekennzeichnet, dass** die Reinigung durch Ultraschallbehandlung erreicht wird.

9. Verfahren nach Anspruch 8, **dadurch gekennzeichnet, dass** die Ultraschallbehandlung mindestens 15 Minuten lang durchgeführt wird.

## Revendications

1. Procédé de carburation de surfaces d'implants médicaux en alliage de titane médical Ti₆Al₄V, comprenant les étapes de :
a) nettoyage de la surface,
b) mise en place de l'alliage dans un four de carburation sous vide, mise sous vide dudit four de carburation, à une pression de 1 Pa à 200 Pa,
c) chauffage dudit four de carburation jusqu'à une température de 1 000 à 1 450°C et maintien de ladite température constante,
d) introduction de C₂H₂ dans le four de carburation sous vide jusqu'à ce que la pression dans le four atteigne 1 000 à 3 500 Pa,
e) mise sous vide et décharge du gaz résultant de la décomposition du C₂H₂
f) arrêt du chauffage et mise sous vide du four en continu, et
g) extraction de l'alliage de titane du four de carburation.

2. Procédé selon la revendication 1, **caractérisé en ce que** les étapes d) et e) sont répétées.

3. Procédé selon l'une quelconque des revendications précédentes, **caractérisé en ce que** la mise sous vide selon l'étape e) se poursuit jusqu'à ce que la température soit refroidie à au moins 80°C.

4. Procédé selon l'une quelconque des revendications précédentes, **caractérisé par** h) le polissage de la surface.

5. Procédé selon l'une quelconque des revendications précédentes, **caractérisé en ce que** l'alliage de titane médical est une tête du fémur pour une arthroplastie de la hanche ou une articulation artificielle pour une arthroplastie du genou.

6. Procédé selon l'une quelconque des revendications précédentes, **caractérisé en ce que** la surface obtenue est une céramique TiC.

7. Procédé selon l'une quelconque des revendications précédentes, **caractérisé en ce que** la surface est nettoyée avec une solution d'acétone.

8. Procédé selon l'une quelconque des revendications précédentes, **caractérisé en ce que** le nettoyage est obtenu par traitement ultrasonore.

9. Procédé selon la revendication 8, **caractérisé en ce que** le traitement ultrasonore est réalisé pendant au moins 15 minutes.
